Europäisches Patentamt

European Patent Office

Office européen des brevets

(11) Publication number: **0 296 285 B1**

# EUROPEAN PATENT SPECIFICATION

(45) Date of publication of patent specification: **29.07.92**    (51) Int. Cl.⁵: **B01J  37/00**, B01J 23/78, C07C 15/46, C07C 5/333

(21) Application number: **87305647.7**

(22) Date of filing: **24.06.87**

(54) **A dehydrogenation catalyst having improved moisture stability and a process for making the same.**

(43) Date of publication of application:
**28.12.88 Bulletin  88/52**

(45) Publication of the grant of the patent:
**29.07.92 Bulletin  92/31**

(84) Designated Contracting States:
**BE DE FR GB IT NL**

(56) References cited:
**EP-A- 0 093 518**
**EP-A- 0 181 999**
**FR-A- 1 220 381**
**FR-A- 2 226 256**
**GB-A- 1 342 957**

(73) Proprietor: **THE DOW CHEMICAL COMPANY**
**2030 Dow Center Abbott Road P.O. Box 1967**
**Midland Michigan 48640-1967(US)**

(72) Inventor: **Moore, Stanley E.**
**113 Hickory**
**Lake Jackson Texas 77566(US)**

(74) Representative: **Burford, Anthony Frederick et al**
**W.H. Beck, Greener & Co. 7 Stone Buildings Lincoln's Inn**
**London WC2A 3SZ(GB)**

## Description

Promoted iron oxide catalysts have been known for many years as dehydrogenation catalysts. They are especially useful in the manufacture of styrene by the dehydrogenation of ethylbenzene. Most of the catalysts now in commercial use employ minor amounts of promoters, e.g. salts or oxides of chromium, manganese, bismuth, tungsten, or molybdenum, with chromium being the preferred minor component, together with compound of potassium, e.g. potassium oxide or carbonate. The last component gives the catalyst a self-regenerative property enabling its use for long periods of time without significant loss in activity. More recent improvements include the incorporation of minor amounts of vanadium, cerium, and of modifiers (such as carbon black or graphite and methyl cellulose) which affect the pore structure of the catalysts. None of these improvements has dealt with the physical integrity of the catalysts. Improved stability to moisture is desirable while maintaining high activity and high yield.

Catalyst life of dehydrogenation catalysts is often dictated by the pressure drop across a reactor, the increase of which lowers both the yield and conversion to the desired vinyl aromatic. For this reason, the physical integrity of the catalyst is of major importance.

In recent years catalysts with higher amounts of potassium have been used, e.g. 20 percent or more, up to 48 percent of potassium calculated as the oxide. Thus, in U.S. A 4,503,163, catalysts are disclosed which contain 13 to 48 percent and preferably 27 to 41 percent by weight of a potassium promoter compound, calculated as potassium oxide. Such catalysts are self regenerative catalysts which perform well at lower steam to oil ratios, e.g. ratios of <2/1. The economic advantages of using less steam are obvious. Associated with the higher amounts of potassium used in such catalysts has been an increase in physical degradation due to moisture and "wet steam" during start-up conditions and plant upsets. This physical degradation can cause increased pressure drop due to coking, plugging, and reduction of the void volume in the catalyst bed.

Calcination processes being practiced by the industry at present are either low temperature calcination processes producing catalyst which can operate at low steam to oil ratios, but is not moisture stable, or high temperature calcination processes producing catalysts which are moisture stable but cannot operate adequately at low steam to oil ratios.

It would be desirable if a catalyst could be prepared which had both high activity and resistance to moisture and which can operate at low steam to oil ratios. A method has now been discovered which will provide such a catalyst.

Dehydrogenation catalysts of the present invention are no different with respect to chemical composition and are made from the same constituents as those of the prior art. Thus, red or yellow iron oxides can be used and the promoter materials are likewise those known to the art. Promoter compounds incorporated into the catalyst are $K_2Cr_2O_7$, $K_2CO_3$, $Cr_2O_3$, $V_2O_5$ and $Co(OH)_2$ or other compounds of these metals such as, for example, acetates, nitrates and oxalates which are reducible to their oxides. Other metal compounds which may be added as promoters include compounds of aluminum, cadmium, cerium, magnesium, manganese and nickel, provided they can be calcined to the oxide.

In addition to the catalytic components, binders and porosity agents are employed in the mixture from which the catalyst is made. A refractory cement is usually employed as a binder and carbon-containing materials, such as graphite and methyl cellulose, may be used to provide porosity which is obtained when they are pyrolyzed during the manufacturing process.

The process of the present invention is a two-step calcination process. It employs tablets or pellets of the catalytic components, which materials have been dried subsequent to being pelletted or tabletted. The first step in the process is conducted at a temperature in the range of from 250° to 600°C in which the carbonaceous materials (porosity agents) are burned out. Temperatures below 250°C will not completely oxidize the carbonaceous materials and temperatures above 600°C can cause the reduction of iron oxide as the organics burn. Above 350°C it becomes necessary to purge the catalyst environment with air in order to prevent the reduction of iron.

The chemical reactions taking place during the first calcination step can be described as follows:

(1) Cellulose pyrolization

$$O_2 \ + \ C_xH_yO_z \rightarrow CO_2 \ + \ H_2O$$

(2) Graphite or carbon pyrolization

$$C \ + \ O_2 \rightarrow CO \ + \ CO_2$$

The second step in the calcination process, which can follow the first without any cooling, calcines the catalyst at temperatures in the range of from 700°C to 800°C. During this calcination step, the carbonate or carbonates present are converted to the corresponding oxides.

The chemical reactions and transformations occurring during the second calcination step can be described as:

(3) Potassium carbonate decomposition

$$K_2CO_3 \rightarrow K_2O + CO_2$$

(4) Potassium ferrite formation

$$K_2O + Fe_2O_3 \rightarrow K_2Fe_2O_4 + KFe_5O_8$$

(5) Clay dehydroxylation and dehydration

(6) Potassium silicate formation from potassium-clay interaction

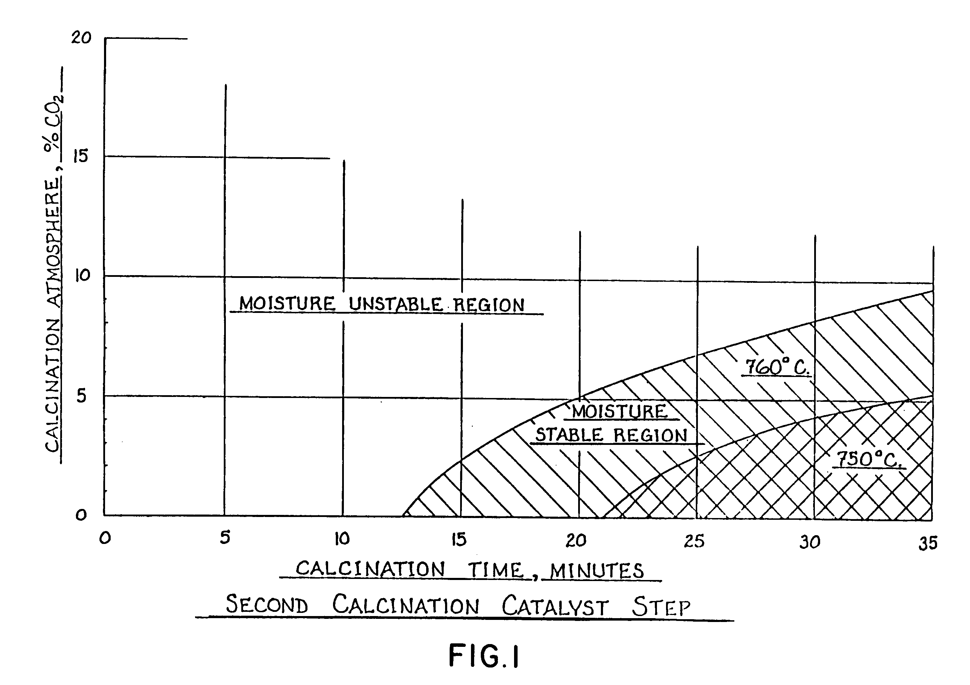

Removal of the evolved $CO_2$ from the vicinity of the catalyst during the second step by providing a gas flow over the catalyst, e.g. air, shortens the time needed for carbonate decomposition. The shorter the time required for this second step, the more active the resulting catalyst. This shorter time also provides a more moisture stable catalyst with no loss in activity over the moisture unstable catalyst containing the same catalytic components. The Figure shows the relationship between the percent $CO_2$ in the atmosphere above the catalyst during the calcination step and the time at that temperature required to provide a moisture-stable catalyst. While only two temperatures are shown, the higher temperatures should provide an area of stability delineated by a line paralleling the two lower temperature indicia.

The preferred temperature for the first step is within the range of from 350° to 600°C and for the second step from 750° to 790°C.

The length of time necessary for the calcination in each step will vary with the temperature employed, the higher temperatures requiring shorter times. Generally, however, from 30 minutes to 4 hours is sufficient time for the first step and from 10 minutes to 60 minutes for the second step. The amount of $CO_2$ evolved and its rate of removal affects the time needed to complete this step. The time is shortened if the gases (oxidation products and $CO_2$) are simultaneously removed as they evolve. This is especially true in the second step where there is an equilibrium existing between the carbonate in the catalyst and the $CO_2$ in the atmosphere surrounding the catalyst.

The present invention will now be more fully described with reference to the accompanying examples.

Examples 1 to 8

In a representative example of making the catalyst, $K_2CO_3$ is provided in an aqueous solution to which $K_2Cr_2O_7$ is added. This solution is then mixed into a dry blend of $Fe_2O_3$, graphite, cement, clay and methyl cellulose to form a slurry which is fed to a spray drier. The slurry normally contains 50 percent water, but may contain as little as 20 percent, or as much as 70 percent. A preferred water content is from 40 to 60 percent. The spray dried material, which is fed to a tabletting machine, usually contains <1 percent water. Details of such a process are described in U.S.-A-4,139,497.

Alternatively, the mixture can be fed as a paste directly to a pelleting machine, in which case the water content is from 8 to 12 percent. This paste is formed as a slurry with 20 to 30 percent water and subsequently dried to an extrudable mass which contains the above correct water content. This alternate process is described more fully in U.S.-A-3,849,339 and U.S.-A-3,703,593.

The present invention is directed to a method of calcining such pelleted or tabletted catalyst precursors. Table I shows the composition of a number of different catalysts before calcining.

The calcining of the compositions of Examples 1-7 in Table I was done by heating the catalyst pellets in a furnace up to 600°C over a period of approximately one-half hour. The pellets were held at this temperature for 2 hours, then the temperature in the furnace was raised to 760°C over a period of approximately one-quarter hour and then maintained at that temperature for one-half hour. The catalyst of Example 8 was calcined in the same way except that the temperature of the first step was 595°C and that of the second step was 790°C.

3

## TABLE I

### Components (% Wt.)

| Example No. | Fe$_2$O$_3$ | K$_2$CO$_3$ | K$_2$Cr$_2$O$_7$ | Cement | Graphite | Clay | Cellulose | Other |
|---|---|---|---|---|---|---|---|---|
| 1 | 46.7 | 34.6 | 0.9 | 4.4 | 6.0 | 4.5 | 0.9 | 2.0 ZrO$_2$ |
| 2 | 45.7 | 33.9 | 0.8 | 4.2 | 4.2 | 8.4 | 0.8 | 2.0 KHSO$_4$ |
| 3 | 44.9 | 33.2 | 0.8 | 4.1 | 4.1 | 8.3 | 0.8 | 3.8 H$_3$PO$_4$ |
| 4 | 45.7 | 33.9 | 0.8 | 4.2 | 4.2 | 8.4 | 0.8 | 2.0 KH$_2$PO$_4$ |
| 5 | 46.5 | 34.5 | 0.9 | 4.3 | 4.3 | 0.0 | 0.9 | 8.6 TiO$_2$ |
| 6 | 48.7 | 36.0 | 0.9 | 4.5 | 4.5 | 0.0 | 0.9 | 4.5 K$_2$B$_4$O$_7$ |
| 7 | 34.7 | 34.2 | 0.9 | 4.5 | 4.5 | 8.9 | 0.9 | 11.4 MgO |
| 8 | 46.5 | 34.7 | 0.9 | 4.3 | 4.3 | 8.5 | 0.9 | --- |

The catalysts were tested for moisture stability and for their effectiveness as dehydrogenation catalysts as follows:

## DEHYDROGENATION REACTION

The catalysts shown in Table I, after completing the two-step calcination, were placed in a laboratory reactor made of 1 inch (25.4 mm) OD pipe, 36 inch (915 mm) long and wrapped with beaded electrical heaters and insulated. A preheater section containing an inert column packing material preceded the catalyst bed. This assured that the ethylbenzene and water were in the vapor phase, mixed well and heated to the reaction temperature prior to contact with the catalyst. A volume of 70 ml of catalyst was provided to the reactor. Different weight ratios of steam to ethylbenzene (steam/oil, or S/O) are indicated for Examples 1-8 in Table II and Example 9 in Table III. Each catalyst was allowed a minimum of 14 days operation as a break-in period before conversions and yields were recorded. Each of these catalysts gave adequate conversion and selectivity for the dehydrogenation of ethylbenzene to styrene.

MOISTURE TEST

The moisture stability of the catalysts was measured by the following method: Twenty pellets or tablets were placed in deionized water (sufficient to cover) and left standing for thirty minutes after which they were visually inspected for retention of shape and form. The number retaining their original form was noted and recorded as a percentage of the total used in the test, i.e. 20 tablets. For any given catalyst to pass the moisture test it must maintain 90 percent or greater of its original form.

Table II shows the results of moisture stability tests of the catalyst compositions from Table I after the first and second calcination steps along with the activity and selectivity as determined using the dehydrogenation reaction on each of these catalysts determined after the second calcination. The activity of the catalyst is indicated by the temperature necessary to achieve a conversion of 50 percent, i.e. the lower the temperature, the higher the activity.

TABLE II

| CATALYST ACTIVITY AND MOISTURE STABILITY | | | | | |
|---|---|---|---|---|---|
| EXAMPLE | MOISTURE TEST | | 50 PERCENT CONVERSION | | S/O** |
| | Step 1 | Step 2 | Temp. °C | % Selectivity | |
| 1 | Failed | Passed | 588 | 93.6 | 1.4 |
| 2 | Failed | Passed | 589 | 93.8 | 1.0 |
| 3 | Failed | Passed | 601 | 93.2 | 1.0 |
| 4 | Failed | Passed | 594 | 93.7 | 1.0 |
| 5* | Failed | Passed | 605 | 95.0 | 1.2 |
| 6 | Failed | Passed | 603 | 94.7 | 1.4 |
| 7 | Failed | Passed | 612 | 95.0 | 1.2 |
| 8 | Failed | Passed | 593 | 94.2 | 1.0 |

*40 percent Conversion

**S/O is the weight ratio of steam to oil, i.e. water to ethylbenzene.

Example 9

The catalyst of Example 8 was calcined for 2 hours at 595°C, but at different timed intervals for the second step at a temperature of 790°C. Table III shows the effect of time on the activity and selectivity of the catalyst in dehydrogenating ethylbenzene and the moisture stability of the catalyst. The temperature is that required to give 50 percent conversion.

EP 0 296 285 B1

TABLE III

| TIME OF SECOND CALCINATION | MOISTURE TEST After Step 2 | 50% CONVERSION | | S/O |
|---|---|---|---|---|
| | | Temp. °C | % Selectivity | |
| 12 min. | Failed | 586 | 94.0 | 1.0 |
| 16 min. | Passed | 596 | 93.6 | 1.0 |
| 20 min. | Passed | 598 | 93.7 | 1.0 |

Example 10 and Comparative Example A (one-step process)

Catalyst pellets having the composition of Example 8 (Table I) were heated to the desired calcination temperature over a period of approximately one-half hour and then maintained at that temperature for different periods of time. The temperatures and lengths of time of calcination together with results of the moisture and dehydrogenation tests are given in Table IV. The weight ratio of steam to hydrocarbon, i.e. water to ethylbenzene, was 1.0. Since one of the catalysts failed the moisture test, it was not tested for dehydrogenation.

TABLE IV

| CALCINATION | | MOISTURE TEST (Single Step) | 50% CONVERSION | |
|---|---|---|---|---|
| Temp. (°C) | Time (hrs.) | | Temp. (°C) | % Select. |
| 700 | 3 | Failed | --- | --- |
| 700 | 4 | Passed | 591 | 93.3 |
| 800 | 1-1/2 | Passed | 605 | <93.0 |

Comparing the results in Example 10 with those of Example 8 in Table II, one sees that while the calcination using the two steps of the invention requires substantially the same temperature for 50% conversion as that using one step at 700°C for four hours, the selectivity is nearly one percentage point difference. This is a significant advantage for the process of the invention. The difference is even more apparent when the comparison is made with the catalyst employing one step calcination at 800°C. Here, both temperatures at conversion 50 percent and selectivity show an advantage for the process of the invention.

Example 11 and Comparative Example B

In yet another experiment, the percent $CO_2$ in the atmosphere above the catalyst being calcined was measured during the second step at temperatures of 750°C and 760°C during timed intervals and moisture stability determined for catalysts calcined for the different lengths of time and temperatures. The results are shown in the Figure. Note that at 0 percent $CO_2$, it was necessary to heat the catalyst for 12.5 minutes at 760°C, but a longer time of 21 minutes was required at 750°C. At 5 percent $CO_2$, 20 minutes heating was required at 760°C, but between 32 and 33 minutes was required at 750°C to obtain a moisture stable catalyst. This shows the importance of removing the $CO_2$ as it is evolved from the catalyst and of keeping the $CO_2$ concentration in the atmosphere above the calcined catalyst as low as possible.

A test of two commercial scale catalysts having the same composition was conducted, one being calcined at 597°C (Comparative Sample B) and the other at 595°C, followed by further calcining at 785°C in accordance with the process of the present invention (Example 11). Results are shown in Table V:

TABLE V

| Property | Comparative Sample B | Example 11 |
|---|---|---|
| Average Crush Strength | 29.6 psi (0.2 MPa) | 33.4 psi (0.23 MPa) |
| % Attrition | 0.19 | 0.76 |
| Water Stability* | 0% | 100% |
| Average Reactor Temperature (°C) | 597 | 593 |
| % Conversion | 50 | 50 |
| % Selectivity | 94.0 | 94.2 |

*The water stability test was performed as previously described.

The sacrifice of some degree of attrition in a catalyst having high water stability is acceptable since the breaking up of pellets due to water is much more severe than that caused by handling the catalyst in shipping and/or reactor loading operations.

**Claims**

1. A process for making an iron oxide-containing dehydrogenation catalyst wherein an alkali metal carbonate is employed as a promoter, organic materials are employed as porosity control agents and the constituents are combined, formed into a particulate mass and calcined at temperatures sufficient to oxidize the organic materials and convert the carbonate to an oxide characterized in that the calcining process is conducted in two steps, wherein said particulate mass is heated in a first step to a temperature between 250°C and 600°C sufficient to oxidize said organic materials and thereafter heated in a second step to a temperature between 700°C and 800°C sufficient to convert substantially all carbonates present to the corresponding oxides.

2. A process as claimed in Claim 1, wherein the porosity control agent is graphite or methyl cellulose.

3. A process claimed in Claim 1 or Claim 2 wherein the products of oxidation in the first step and/or the carbon dioxide of the second step are removed simultaneously as they are evolved from said mass.

4. A process as claimed in Claim 3, wherein the removal of evolved products of oxidation is accomplished by a flow of gas over said mass.

5. A process as claimed in Claim 4, wherein the flowing gas is air.

6. A process as claimed in any one of the preceding claims, wherein the temperature of steps 1 and/or 2 are 350° to 600°C and 750° to 790°C, respectively.

7. A process as claimed in any one of the preceding claims, wherein the time of calcination is from 30 minutes to 4 hours for step 1 and from 10 to 60 minutes for step 2.

8. A moisture-stable iron oxide-containing dehydrogenation catalyst preparable by a process as claimed in any one of the preceding claims.

9. A process for preparing styrene by dehydrogenation of ethyl benzene in the presence of a promoted iron oxide dehydrogenation catalyst, characterised in that the catalyst is as claimed in claim 8.

**Revendications**

1. Procédé pour la préparation d'un catalyseur de déshydrogénation contenant un oxyde de fer, dans lequel on utilise un carbonate de métal alcalin en tant qu'activeur, on utilise des substances organiques en tant qu'agents de réglage de la porosité, et les constituants sont réunis, mis sous forme d'une masse particulaire et calcinés à des températures suffisantes pour oxyder les substances organiques et convertir le carbonate en un oxyde, caractérisé en ce que le processus de calcination est effectué en deux étapes, ladite masse particulaire étant chauffée, dans une première étape, à une température comprise entre 250 et 600°C, suffisante pour oxyder lesdites substances organiques, et étant ensuite chauffée, dans une seconde étape, à une température comprise entre 700 et 800°C, suffisante pour convertir pratiquement tous les carbonates présents en les oxydes correspondants.

2.  Procédé selon la revendication 1, dans lequel l'agent de réglage de la porosité est le graphite ou la méthylcellulose.

3.  Procédé selon la revendication 1 ou 2, dans lequel les produits d'oxydation dans la première étape et/ou le dioxyde de carbone de la seconde étape sont éliminés au fur et à mesure qu'ils se dégagent de ladite masse.

4.  Procédé selon la revendication 3, dans lequel l'élimination des produits d'oxydation dégagés est effectuée par un courant de gaz circulant sur ladite masse.

5.  Procédé selon la revendication 4, dans lequel le gaz circulant est l'air.

6.  Procédé selon l'une quelconque des revendications précédentes, dans lequel la température de l'étape 1 et la température de l'étape 2 sont de 350 à 600°C et de 750 à 790°C, respectivement.

7.  Procédé selon l'une quelconque des revendications précédentes, dans lequel la durée de calcination est de 30 minutes à 4 heures pour l'étape 1 et de 10 à 60 minutes pour l'étape 2.

8.  Catalyseur de déshydrogénation contenant un oxyde de fer, résistant à l'humidité, préparable par un procédé tel que revendiqué dans l'une quelconque des revendications précédentes.

9.  Procédé pour la préparation du styrène par déshydrogénation de l'éthylbenzène en présence d'un catalyseur de déshydrogénation à base d'oxyde de fer activé, caractérisé en ce que le catalyseur est tel que revendiqué dans la revendication 8.

**Patentansprüche**

1.  Verfahren zur Herstellung eines Eisenoxid enthaltenden Dehydrierungskatalysators, wobei man ein Alkalimetallcarbonat als Aktivator, organische Materialien als Kontrollmittel für die Porosität verwendet und die Bestandteile vereinigt, zu einer teilchenförmigen Masse formt und bei Temperaturen kalziniert, die ausreichend sind, um die organischen Materialien zu oxidieren und das Carbonat in ein Oxid zu überführen, **dadurch gekennzeichnet,** daß der Kalzinierungsprozeß in zwei Schritten durchgeführt wird, wobei man die teilchenförmige Masse in einem ersten Schritt auf eine Temperatur zwischen 250°C und 600°C, die ausreichend ist, um die organischen Materialien zu oxidieren, erhitzt und danach in einem zweiten Schritt auf eine Temperatur zwischen 700°C und 800°C, die ausreichend ist, im wesentlichen alle vorhandenen Carbonate in die entsprechenden Oxide zu überführen, erhitzt.

2.  Verfahren nach Anspruch 1, worin das Kontrollmittel für die Porosität Graphit oder Methylcellulose ist.

3.  Verfahren nach Anspruch 1 oder 2, worin die Oxidationsprodukte im ersten Schritt und/oder das Kohlendioxid des zweiten Schritts simultan mit dem Entweichen aus der Masse entfernt werden.

4.  Verfahren nach Anspruch 3, worin die Entfernung der entweichenden Oxidationsprodukte durch einen Gasstrom über der Masse bewirkt wird.

5.  Verfahren nach Anspruch 4, worin das strömende Gas Luft ist.

6.  Verfahren nach einem der vorhergehenden Ansprüche, worin die Temperatur der Schritte 1 und/oder 2 350° bis 600°C bzw. 750° bis 790°C ist.

7.  Verfahren nach einem der vorhergehenden Ansprüche, worin die Kalzinierungszeit von 30 Minuten bis 4 Stunden für Schritt 1 und von 10 bis 60 Minuten für Schritt 2 ist.

8.  Feuchtigkeitsbeständiger, Eisenoxid enthaltender Dehydrierungskatalysator, erhältlich durch ein Verfahren nach einem der vorhergehenden Ansprüche.

9. Verfahren zur Herstellung von Styrol durch Dehydrierung von Ethylbenzol in Gegenwart eines aktivierten Eisenoxid-Dehydrierungskatalysators, **dadurch gekennzeichnet,** daß der Katalysator gemäß Anspruch 8 ist.

SECOND CALCINATION CATALYST STEP

FIG.I

EP 0 296 285 B1